(19) Europäisches Patentamt
European Patent Office
Office européen des brevets

(11) Publication number: **0 067 641**
**A2**

# (12) EUROPEAN PATENT APPLICATION

(21) Application number: 82302929.3

(22) Date of filing: 08.06.82

(51) Int. Cl.³: C 07 C 101/28
C 07 D 213/38, C 07 D 333/20
C 07 D 307/52, A 61 K 31/195
A 61 K 31/395

(30) Priority: 17.06.81 GB 8118607

(43) Date of publication of application:
22.12.82 Bulletin 82/51

(84) Designated Contracting States:
CH DE FR GB IT LI NL

(71) Applicant: BEECHAM GROUP PLC
Beecham House Great West Road
Brentford Middlesex(GB)

(72) Inventor: Ferres, Harry
20 Garlichill Road
Epsom Surrey(GB)

(74) Representative: Cresswell, Thomas Anthony et al,
European Patent Attorney Beecham Pharmaceuticals
Great Burgh Yew Tree Bottom Road
Epsom Surrey KT18 5XQ(GB)

## (54) Amino acids.

(57) A compound of formula (III):

$$Z - CH_2 - NH - \underset{\alpha}{\overset{R^1}{\overset{|}{CH}}} - CO_2H \qquad (III)$$

pharmaceutically acceptable salt, ester amide or bioprecursors thereof,

wherein $R^1$ is optionally substituted aryl, aralkyl, aromatic heterocyclic or aromatic heterocyclylalkyl group, and

Z is a naphthyl group,

optionally substituted with one or more groups selected from $C_{1-6}$ alkyl, halogen, hydroxy or trifluoromethyl,

or Z is a 5 or 6 membered heterocyclic group containing one or more nitrogen, oxygen or sulphur atoms, with at least one of the hetero- atoms being attached to the same carbon atom as the methylene group, and optionally substituted with $C_{1-6}$ alkyl, halogen, hydroxy or trifluoromethyl, with the proviso that when $R^1$ is benzyl and Z is a naphthyl group, the compound of formula (III) is not in the DL-form.

EP 0 067 641 A2

"N-SUBSTITUTED α-AMINO ACIDS, PROCESSES FOR MAKING THEM AND PHARMACEUTICAL COMPOSITIONS CONTAINING THEM"

This invention relates to a class of N-substituted α-amino acids which are potentially useful in the treatment of atherosclerosis.

The invention also relates to processes for the preparation of such compounds and pharmaceutical compositions comprising them.

N-Substituted α-amino acids, in particular, N-benzyl-α-amino acids, have been used extensively as protected derivatives for use in peptide synthesis; however, relatively few simple amino acid derivatives have been investigated in respect of bioactivity.

The N-substituted-DL-amino acids of formula (I) are known from J. Pharm. Soc. Japan, 1978, 98, (7) 869-879.

$$R^a - NH - \underset{|}{\overset{CH_2 - R}{}}CH - CO_2H \qquad (I)$$

wherein R is phenyl and $R^a$ is benzyl, 4-chlorobenzyl, 2-hydroxybenzyl, $\alpha$-$C_{10}H_7CH_2$-, $\beta$-$C_{10}H_7CH_2$- and cyclo $C_6H_{11}$-, and wherein R is hydrogen and $R^a$ is benzyl and 4-chlorobenzyl.

Biological tests, including antibacterial activity and the action on central nervous, circulation and muscular systems were carried out; however, the publication discloses that none of the compounds showed much activity.

Our Published European Application No. 0031653 discloses compounds of the formula (II)

$$R^2 - CH_2 - NH - \underset{\alpha}{\overset{R^1}{\overset{|}{CH}}} - CO_2H \qquad (II)$$

wherein $R^1$ represents an optionally substituted aryl, aralkyl, aromatic heterocyclic or aromatic heterocyclyl-alkyl group, and $R^2$ represents a phenyl group optionally substituted with $C_{1-6}$ alkyl, halogen, hydroxy, trifluoromethyl or methylenedioxy. These compounds promote a more favourable composition of lipoproteins in the bloodstream, i.e. they are either especially hyperalphalipoproteinaemic [i.e. raise the blood levels of high-density lipoproteins (HDL)], with some hypobetalipoproteinaemic behaviour [i.e. lower blood levels of the low-density lipoproteins (LDL) and/or the very low-density lipoproteins (VLDL)] or exert both effects.

We have now discovered a further class of N-substituted amino acids, structurally different from those of formula (II), which also possess properties similar to those of the compounds of formula (II).

Accordingly, the present invention provides an α-amino acid derivatives of formula (III) or a pharmaceutically acceptable salt, ester, amide or bioprecursor thereof:

$$Z - CH_2 - NH - \underset{\alpha}{\overset{\overset{R^1}{|}}{CH}} - CO_2H \qquad (III)$$

wherein $R^1$ is optionally substituted aryl, aralkyl, aromatic heterocyclic or aromatic heterocyclylalkyl group, and
Z is a naphthyl group,

optionally substituted with one or more groups selected from $C_{1-6}$ alkyl, halogen, hydroxy or trifluoromethyl,

or Z is a 5 or 6 membered heterocyclic group containing one or more nitrogen, oxygen or sulphur atoms, with at least one of the hetero- atoms being attached to the same carbon atom as the methylene group, and optionally substituted with $C_{1-6}$ alkyl, halogen, hydroxy or trifluoromethyl, with the proviso that when $R^1$ is benzyl and Z is a naphthyl group, the compound of formula (III) is not in the DL-form.

Preferred heterocyclic groups for Z are pyridinyl, thiophenyl, furanyl, pyrimidinyl, isothiazolyl, isoxazolyl pyrazinyl, thiazolyl and oxazolyl.

Preferably $R^1$ represents phenyl or benzyl optionally substituted with halogen, lower alkyl or hydroxyl, or a 5 or 6 membered heterocyclic group containing one or more

nitrogen, oxygen or sulphur atoms and optionally substituted with lower alkylene, or a bicyclic aromatic heterocyclic group containing one or more nitrogen, oxygen or sulphur atoms.

Compounds of formula (III) contain at least one asymmetric carbon atom (marked by the α) and hence may exist in two optically active forms, the D-form and the L-form or as an optically inactive racemate. Included within the scope of this invention, therefore, are the substantially pure optical isomers, the racemate and mixtures thereof.

Preferably the compounds (III) are in the substantially pure L-form.

The term 'pharmaceutically acceptable bioprecursor' used above requires some explanation. It is, of course, common practice in pharmaceutical chemistry to overcome some undesirable physical or chemical property of drug by converting the drug into a chemical derivative which does not suffer from that undesirable property, but which, upon administration to an animal or human being, is converted back to the parent drug. For example, if the drug is not well absorbed when given to the animal or patient, by the oral route, it may be possible to convert the drug into a chemical derivative which is well absorbed and which in the serum or tissues is reconverted to the parent drug. For example, if the drug is unstable in solution, it may be possible to prepare a chemical derivative of the drug which is stable and may be administered in solution, but which is reconverted in the body to give the parent drug.

The pharmaceutical chemist is well aware of the possibility of overcoming intrinsic deficiencies in a drug by chemical modifications which are only temporary and are reversible upon administration to the animal or patient.

For the purpose of this specification the term 'pharmaceutically acceptable bioprecursor' of a drug means a compound having structural formula different from the drug which nonetheless, upon administration to an animal or human being, is converted in the patient's body to the drug.

Suitable pharmaceutically acceptable bioprecursors include *in vivo* hydrolysable ester groups.

Examples of groups which hydrolyse readily in the body to produce the parent acid include acyloxyalkyl groups such as acetoxymethyl, pivaloyloxymethyl, α-acetoxyethyl, α-acetoxybenzyl and α-pivaloyloxyethyl groups; alkoxycarbonyloxyalkyl groups, such as ethoxycarbonyloxymethyl and α-ethoxycarbonyloxyethyl; dialkylaminoalkyl groups such as dimethylaminomethyl, dimethylaminoethyl, diethylaminomethyl or diethylaminoethyl; and lactone groups such as phthalidyl.

Suitable carboxylic acid salts of compound (III) include alkali metal salts such as lithium, sodium and potassium, other metal salts such as barium, calcium, aluminium or ammonium or substituted ammonium salts.

Suitable acid addition salts of compound (III) include inorganic salts such as the sulphate, nitrate, phosphate and borate, hydrohalides such as the hydrochloride, hydrobromide and hydroiodide, and organic acid addition salts such as acetate, oxalate, tartrate, maleate, citrate, succinate, benzoate, ascorbate, methanesulphonate, and *p*-toluenesulphonate.

Preferred esters of the compounds of formula (III) are lower alkyl esters, particularly the methyl ester.

The compounds of this invention may be prepared by reacting an amino compound of formula (IV):

$$H_2N - \underset{\alpha}{\overset{\overset{\displaystyle R^1}{|}}{CH}} - CO_2R^x \qquad (IV)$$

wherein $R^1$ is as defined with respect to formula (III) and any reactive groups therein may be protected, and $R^x$ is hydrogen or a carboxy blocking group, with a carbonyl compound of formula (V):

$$Z - CHO \qquad (V)$$

wherein Z is as defined with respect to formula (III) and any reactive groups may be protected, followed by reduction of the resulting Schiff's base with a suitable hydride reducing agent, or hydrogen in the presence of a catalyst; and thereafter, if necessary, carrying out one or more of the following steps:

i) removing any carboxyl-blocking group $R^x$;
ii) removing any protecting groups on $R^1$ or Z;
iii) converting the product into a pharmaceutically acceptable salt, ester, amide or a pharmaceutically acceptable bioprecursor.

Suitable carboxyl-blocking derivatives for the group $-CO_2R^x$ in formula (III) include salts and ester derivatives of the carboxylic acid. The derivative is preferably one which may readily be cleaved at a later stage of the reaction. Suitable salts include tertiary amine salts, such as those with tri-lower alkylamines, N-ethylpiperidine, 2,6-lutidine, pyridine, N-methylpyrrolidine, dimethylpiperazine.

Suitable ester-forming carboxyl-blocking groups are those which may be removed under conventional conditions. Such groups for $R^x$ include methyl, ethyl, benzyl, p-methoxybenzyl, 2,4,6-trimethylbenzyl, 3,5-di-t-butyl-4-hydroxybenzyl, benzolymethyl, p-nitrobenzyl, 4-pyridylmethyl, 2,2,2-trichloroethyl, 2,2,2-tribromoethyl, t-butyl, t-amyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, p-toluenesulphonylethyl, methoxymethyl, a silyl, stannyl or phosphorus-containing group, such as described above, or an in vivo hydrolysable ester radical such as defined above.

The carboxyl group may be regenerated from any of the above esters by usual methods appropriate to the particular $R^x$ group, for example, acid and base catalysed hydrolysis, or by enzymically catalysed hydrolysis, or by hydrogenation.

The formation of the Schiff's base is carried out in an organic solvent or solvent mixture and, depending upon the stability of the Schiff's base, it may be isolated and purified or partially purified before reduction.

The choice of organic solvent and reaction conditions will be influenced by the solubility and reactivity of the starting materials and the resulting Schiff's base. The reaction is generally carried out in a hydrocarbon or ethereal solvent at a moderate temperature i.e. greater than room temperature, the reflux temperature of the solvent being selected for convenience.

The period for which the reaction is allowed to proceed depends upon the particular starting materials employed. The course of the reaction may be followed by conventional methods such as thin layer chromatography and terminated when an optimum quantity of product is present in the reaction mixture.

Suitable hydride reducing agents for reduction of the Schiff's base include sodium borohydride and borane-trimethylamine complex. Suitable reaction solvents and reaction conditions are discussed in H.O. House "Modern Synthetic Reactions" 2nd Edition, Chapter 2.

Suitable catalysts for use in the catalytic hydrogenation of the Schiff's base include platinum oxide and Raney nickel.

In cases where the Schiff's base is unstable or where isolation of the Schiff's base is undesirable the amino compound of formula (IV) and carbonyl compound of formula (V) may be dissolved or suspended in organic solvent, mixed, and reduced <u>in situ</u> with a hydride reducing agent. Suitable hydride reducing agents include sodium cyanoborohydride which is preferably used in a protic solvent such as an alcohol, in particular methanol.

The compounds of the present invention may also be prepared by reacting a compound of the formula (VI):

$$R^1 - CO - CO_2R^x \qquad (VI)$$

in which $R^1$ is as defined with respect to formula (III) and $R^x$ is as defined with respect to formula (IV) with a compound of formula (VII):

$$NH_2 - CH_2 - Z \qquad (VII)$$

in which Z is as defined with respect to formula (III) followed by reduction of the adduct.

A further process for preparing the compounds of the present invention comprises the reaction of an amino compound of formula (IV) as defined hereinbefore with a compound of formula (VIII):

$$Z - X \qquad (VIII)$$

wherein Z is as defined with respect to formula (III), and any reactive groups may be protected, and X is a halide; and thereafter where necessary carrying out steps (i) - (iii) as described hereinbefore. Preferably X is chloride, bromide or iodide.

The compounds of the present invention may also be prepared by reducing with a hydride reducing agent an N-acyl compound of formula (IX):

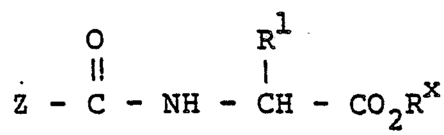

(IX)

wherein $R^1$ and Z are as defined with respect to formula (III), $R^x$ is hydrogen or a carboxyl-blocking group, and thereafter, if necessary, carrying out one or more of steps (i) - (iii) as described hereinbefore.

Suitable hydride reducing agents include the borane dimethylsulphide complex which is conveniently used in ethereal solvents such as tetrahydrofuran.

The N-acyl compounds of formula (IX) may be prepared by reacting an amino compound of formula (VI) as defined hereinbefore wherein the amino group is optionally substituted with a group which permits acylation to take place, and wherein $R^x$ is hydrogen or a carboxyl-blocking group, with an N-acylating derivative of an acid of formula (X):

$$Z.CO_2H \qquad (X)$$

wherein Z is as defined with respect to formula (III) above.

Suitable groups which permit acylation to take place and which are optionally present on the amino group of the starting material of the formula (X) include N-silyl, N-stannyl and N-phosphorus groups, for example trialkylsilyl groups such as trimethylsilyl.

A reactive N-acylating derivative of the acid (X) is employed in the above process. The choice of reactive derivative will, of course, be influenced by the chemical nature of the substituents of the acid.

Suitable N-acylating derivatives include an acid halide, preferably the acid chloride or bromide. Acylation with an acid halide may be affected in the presence of an acid binding agent for example tertiary amine (such as triethylamine or dimethylamiline), an inorganic base (such as calcium carbonate or sodium bicarbonate) or an oxirane, which binds hydrogen halide liberated in the acylation reaction. The oxirane is preferably a $(C_{1-6})$-1,2-alkylene oxide, such as ethylene oxide or propylene oxide.

The acid halide may be prepared by reacting the acid (X) or a salt thereof with a halogenating (e.g. chlorinating or brominating) agent such as phosphorus pentachloride, thionyl chloride or oxalyl chloride.

Alternatively, the N-acylating derivative of the acid (X) may be symmetrical or mixed anhydride. Suitable mixed anhydrides are alkoxyformic anhydrides.

Alternative N-acylating derivatives of acid (X) are the acid azide, or activated esters such as esters with 2-mercaptopyridine, cyanomethanol, *p*-nitrophenol, 2,4-dimitrophenol, thiophenol, halophenols, including pentachlorophenol, monomethoxyphenol or 8-hydroxyquinoline; or amides such as N-acylsaccharins or N-acylphthalimides or an alkylidene iminoester prepared by reaction of the acid (X) with an oxime.

Other reactive N-acylating derivatives of the acid (X) include the reactive intermediate formed by reaction *in situ* with a condensing agent, such as a carbodiimide, for example N,N-diethyl-, dipropyl- or diisopropylcarbodiimide, or N,N'-dicyclohexylcarbodiimide. The condensation reaction is preferably carried out in an organic reaction medium, for example methylene chloride,

dimethylformamide, acetonitrile, alcohol, benzene, dioxan or tetrahydrofuran.

In order to put the compounds of the invention to use as medicinal agents, they are presented as pharmaceutical compositions in a variety of dosage forms.

This invention, therefore, also includes a pharmaceutical composition which comprises a pharmaceutically acceptable carrier or excipient together with a compound of formula (III) or a pharmaceutically acceptable salt, ester or bioprecursor thereof.

The compositions may be formulated for administration by any route, although an oral administration is preferred. The compositions may be in the form of tablets, capsules, powders, granules, lozenges or liquid preparations, such as oral or sterile parenteral solutions or suspensions.

Unit dose presentation forms for oral administration may be tablets and capsules and may contain conventional excipients such as binding agents, for example syrup, acacia, gelatin, sorbitol, tragacanth or polyvinylpyrollidone; fillers, for example lactose, sugar, maize-starch, calcium phosphate, sorbitol or glycine; tabletting lubricants, for example magnesium stearate; disintegrants, for example starch or sodium starch glycollate or pharmaceutically acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use.

Such liquid preparations may contain conventional additives, such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, fractionated coconut oil, oily esters such as glycerine, propylene glycol, or ethyl alcohol; preservatives, for example methyl or propyl _p_-hydroxybenzoate or sorbic acid, and, if desired, conventional flavouring or colouring agents.

For parenteral administration, fluid unit dosage forms are prepared utilising the compound and a sterile vehicle, and depending on the concentration used, can be either suspended or dissolved in the vehicle. In preparing solutions the compound can be dissolved in water for injection and filter sterilised before filling into a suitable vial or ampoule and sealing. Advantageously, adjuvants such as a local anaesthetic, preservative and buffering agents can be dissolved in the vehicle. To enhance the stability, the composition can be frozen after filling into the vial and the water removed under vacuum. Parenteral suspensions are prepared in substantially the same manner except that the compound is suspended in the vehicle instead of being dissolved and sterilisation cannot be accomplished by filtration. The compound can be sterilsed by exposure to ethylene oxide before suspending in the sterile vehicle. Advantageously, a surfactant or wetting agent is included in the composition to facilitate uniform distribution of the compound.

The compositions may contain from 0.1% to 99% by weight, preferably from 10 to 60% by weight, of the active material, depending on the method of administration. The dosage employed for adult treatment will, of course, depend on the dose-response characteristics of the particular active ingredient but will normally be in the range 50 mg to 5 g/day.

The following Examples illustrate the preparation of compounds of this invention.

EXAMPLE 1

Preparation of N-[2-(5-chloro)thienyl]methyl-DL-(4-chlorophenyl)alanine

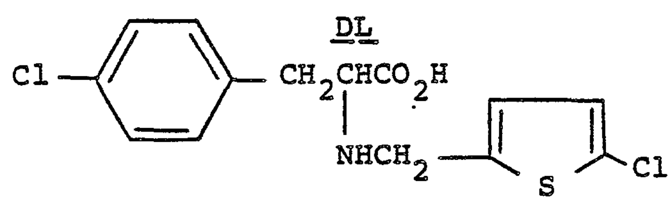

To a mixture of DL-(4-chlorophenyl)alanine (9.98 g, 0.05 mole) and 5-chlorothiophene-2-carboxaldehyde (7.5 g, 0.051 mole) in methanol (200 ml) was added sodium cyanoborohydride (*ca*. 6.3 g, 0.1 mole) portionwise, with stirring at room temperature, over 5 minutes. The reaction mixture was stirred at room temperature overnight and the precipitate was collected and washed with water, followed by methanol. The product was purified by repeated trituration, first with boiling water, then boiling methanol, and finally dried *in vacuo*. N-[2-(5-chloro)thienyl]methyl-DL-(4-chlorophenyl)alanine was obtained as a colourless powder (3.9 g, 24%), mp 220-221°C.

Found: C 51.25; H 3.77; N 4.26; S 9.34; Cl 21.57%
$C_{14}H_{13}Cl_2NO_2S$
Requires: C 50.91; H 3.97; N 4.24; S 9.70; Cl 21.47%

IR (KBr disc): $\nu_{max}$ $1610^{-1}$;
NMR ($CF_3CO_2D$, 90 MHz): δ 3.50 (2H,m); 4.60 (3H,m); 7.20 (6H,m).

The compounds of Examples 2, 3 and 6 were prepared using the method described in Example 1.

EXAMPLE 2

N-(2-Furyl)methyl-DL-(4-chlorophenyl)-alanine

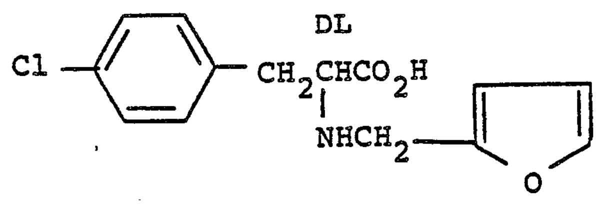

Yield: 27%. Colourless powder, mp 228-229°C.

Found: C 60.45; H 4.94; N 5.03; Cl 12.61%.
$C_{14}H_{14}ClNO_3$ requires C 60.11; H 5.04; N 5.00; Cl 12.67%

IR (KBr disc): $\nu_{max}$ 1610 $cm^{-1}$

NMR ($CF_3CO_2D$, 90 MHz): δ 3.50 (2H,m), 4.56 (3H,m), 6.56 (2H,m), 7.35 (5H,m).

EXAMPLE 3

N-(1-Naphthyl)methyl-L-phenylalanine

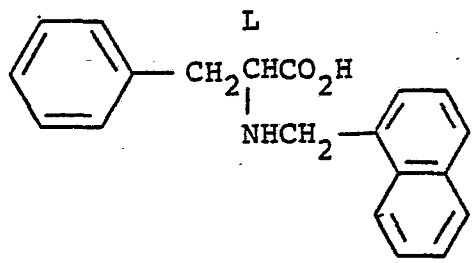

Yield: 24%. Colourless powder, mp 202-202.5°C.
(from ethanol); $[\alpha]_D^{25}$= ca +10° in 6N HCl; acetic acid (1:1).

Found: C 78.10; H 6.63; N 4.55%. $C_{20}H_{19}NO_2$
requires: C 78.66; H 6.27; N 4.59%.

IR (KBr disc): $\nu_{max}$ 1585 $cm^{-1}$.

NMR ($CF_3CO_2D$, 90 MHz): δ 3.40 (2H, 2 x dd), 4.50 (1H,q), 4.78 (1H,d,J=13 Hz), 5.12 (1H,d,J=13 Hz), 6.90 - 8.20 (12H,m).

EXAMPLE 4

Preparation of N-(2-pyridyl)methyl-DL-(4-chlorophenyl) alanine

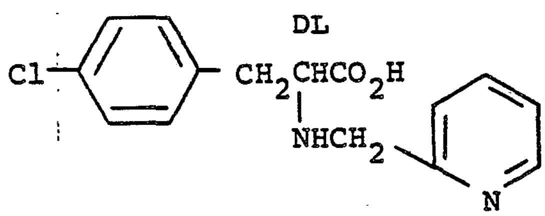

To a mixture of DL-(4-chlorophenyl)alanine (10 g, 0.05 mole) and 2-pyridinecarboxaldehyde (10.8 g, 0.1 mole) in methanol (200 ml) was added sodium cyanoborohydride (8 g, 0.127 mole) portionwise, with stirring at room temperature, over 5 minutes. The reaction mixture was stirred at room temperature for 4 days, filtered, and the fitrate was cautiously acidified to pH 5 with dilute hydrochloric acid. The resulting precipitate was collected, triturated with boiling water, followed by boiling and dried, yielding N-(2-pyridyl)methyl-DL-(4-chlorophenyl) alanine as a colourless powder (1.7 g, 12%) mp 211-212°C.

Found: C 61.95; H 5.41; N 9.76; Cl 12.37%.
$C_{15}H_{15}ClN_2O_2$ requires C 61.96; H 5.20; N 9.64; Cl 12.19%.

IR (KBr disc): $\nu_{max}$ 1610 $cm^{-1}$.

NMR ($CF_3CO_2D$, 90 MHz): $\delta$ 3.55 (2H,m), 4.80 (1H,t), 5.10 (2H,s), 7.30 (4H,m), 8.27 (2H,m), 8.80 (2H,m).

EXAMPLE 5

N-(2-Naphthyl)methyl-L-phenylalanine

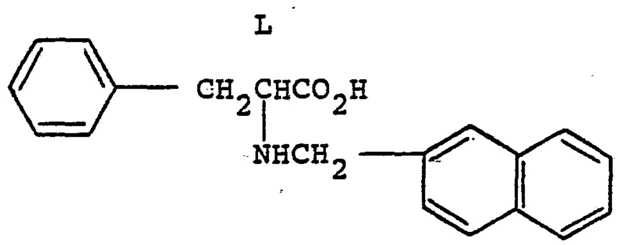

This compound was prepared in the manner described for Example 1, but with methanol-water (2:1) as the reaction medium.

Yield: 40%. Colourless powder, mp 210-211°C;.
(decomp.); $[\alpha]^{25}_{D}$ = 22.1° in 6N HCl - acetic acid (1:1)

(Found: C, 78.33, H, 6.39, N, 4.60% $C_{20}H_{19}NO_2$

Requires: C, 78.66; H, 6.27, N, 4.59%)

$\nu$max (KBr disc) 1620 $cm^{-1}$

$^1$H nmr

($CF_3CO_2D$) $\delta$ 3.40 (2H,m), 4.50 (3H,m), 7.0-8.0 (12H,m).

EXAMPLE 6

N-(2-Furyl)methyl-L-phenylalanine

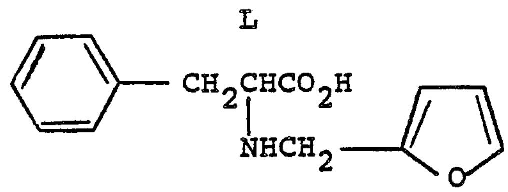

Yield: 38%. Colourless powder, mp 228-230°C;
$[\alpha]^{25}_D$ = 36.2° in 6N HCl-acetic acid (1:1).
(Found: C, 68.50; H, 5.75; N, 5.35% $C_{14}H_{15}NO_3$
Requires: C, 68.55; H, 6.16; N, 5.71%)
$\nu$max. (KBr disc) 1610cm.$^{-1}$

$^1$H nmr ($CF_3CO_2D$)

$\delta$ 3.45 (2H,m), 4.50 (3H,m), 6.50 (2H,br,dd), 7.0-7.7 (6H,m).

BIOLOGICAL DATA

The compounds of the Examples above were tested for their effects on serum lipids *in vivo* using rats made hyperlipidaemic by diet*.

Protocol

Male Sprague-Dawley rats, 80-100 g, were randomised into groups of 8 after 5 - 7 days of feeding on the diet. The compounds were fed to the rats at a concentration of 0.1% of the powdered diet * (ie approximately 100 mg/kg/day), unless otherwise stated. The control groups were fed only the diet. At the end of the experiment, ie after 7 days, the rats were killed and their individual total serum cholesterol and triglyceride measured in mg/100 ml using a Technicon Autoanalyser. The high density lipoprotein (HDL)-cholesterol fraction, which was obtained from the serum by precipitation with heparin-manganous chloride, was measured on four 'pooled' samples from each group.

The results are shown in Table 1 below.

* A semi-synthetic diet rich in sucrose and cholesterol.

TABLE 1

| Compound of Example No. | Percentage difference≠ from Control Values in | | |
|---|---|---|---|
| | Total Serum Cholesterol | Total Serum Triglyceride | High Density lipoprotein (HDL) cholesterol |
| 1 | +16 | -42* | +31* |
| 2 | +61* | -47* | +84* |
| 3 | +62* | -63* | +41* |
| 4 | +78* | -30* | +44* |
| 5 | +17* | -57* | +30* |
| 6 | + 2 | +39 | +26* |

≠ (-) indicates lower and (+) indicates higher than control values.

* Statistically significant result ($p \leq 0.05$).

CLAIMS

1. A compound of formula (III):

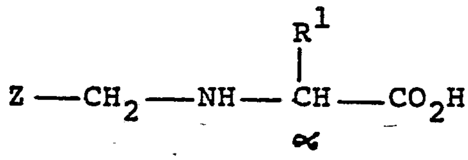

(III)

pharmaceutically acceptable salt, ester amide or bioprecursors thereof,
wherein $R^1$ is optionally substituted aryl, aralkyl, aromatic heterocyclic or aromatic heterocyclylalkyl group, and
Z is a naphthyl group,

optionally substituted with one or more groups selected from $C_{1-6}$ alkyl, halogen, hydroxy or trifluoromethyl,

or Z is a 5 or 6 membered heterocyclic group containing one or more nitrogen, oxygen or sulphur atoms, with at least one of the hetero- atoms being attached to the same carbon atom as the methylene group, and optionally substituted with $C_{1-6}$ alkyl, halogen, hydroxy or trifluoromethyl, with the proviso that when $R^1$ is benzyl and Z is a naphthyl group, the compound of formula (III) is not in the DL-form.

Contd/....

2. A compound as claimed in claim 1, wherein $R^1$ represents phenyl or benzyl optionally substituted with halogen, lower alkyl or hydroxyl, or a 5 or 6 membered heterocyclic group containing one or more nitrogen, oxygen or sulphur atoms and optionally substituted with lower alkylene, or a bicyclic aromatic heterocyclic group containing one or more nitrogen, oxygen or sulphur atoms.

3. A compound as claimed in claim 1 or claim 2, wherein Z is a pyridinyl, thiophenyl, furanyl, pyrimidinyl, isothiazolyl, isoxazolyl, pyrazinyl, thiazolyl, oxazolyl or a naphthyl group.

4. A compound as claimed in any one of claims 1 to 3 wherein $R^1$ is a benzyl group optionally substituted with a halogen atom.

5. A compound as claimed in any one of claims 1 to 4 in the optionally active L-form.

6. A compound as claimed in claim 1 and selected from:

N-[2-(5-chloro)thienyl]methyl-DL-(4-chlorophenyl)alanine,

N-(2-furyl)methyl-DL-(4-chlorophenyl)-alanine,

N-(1-naphthyl)methyl-L-phenylalanine,

N-(2-pyridyl)methyl-DL-(4-chlorophenyl)alanine,

N-(2-naphthyl)methyl-L-phenylalanine,

N-(2-furyl)methyl-L-phenylalanine.

7. A process for preparing a compound of formula (III), as defined in claim 1, which comprises:

a) reacting an amino compound of formula (IV):

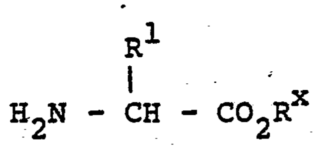

(IV)

wherein $R^1$ is as defined with respect to formula (III), claim 1, and any reactive groups therein may be protected, and $R^x$ is hydrogen or a carboxy blocking group, with a carbonyl compound of formula (V):

$$Z - CHO \qquad (V)$$

wherein Z is as defined with respect to formula (III), claim 1, and any reactive groups may be protected, followed by reduction of the resulting Schiff's base with a suitable hydride reducing agent, or hydrogen in the presence of a catalyst; and thereafter, if necessary, carrying out one or more of the following steps:

i) removing any carboxyl-blocking group $R^x$;

ii) removing any protecting groups on $R^1$ or Z;

iii) converting the product into a pharmaceutically acceptable salt, ester, amide or a pharmaceutically acceptable bioprecursor,

or

b) reacting a compound of the formula (VI):

$$R^1 - CO - CO_2R^x \quad \text{(VI)}$$

in which $R^1$ is as defined with respect to formula (III), claim 1, and $R^x$ is as defined with respect to formula (IV) above, with a compound of formula (VII):

$$NH_2 - CH_2 - Z \quad \text{(VII)}$$

in which Z is as defined with respect to formula (III), as defined in claim 1, followed by reduction of the adduct,

or

c) reacting an amino compound of formula (IV), as defined above, with a compound of formula (VIII):

Z — X

(VIII)

wherein Z is as defined with respect to formula (III), claim 1, and any reactive groups may be proctected, and X is a halide; and thereafter where necessary carrying out steps (i) to (iii), as described above,

or

d) treating an N-acyl compound of formula (IX):

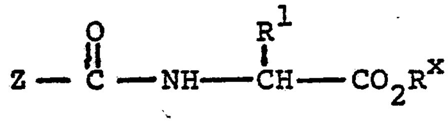

(IX)

wherein Z and $R^1$ are as defined in respect to formula (III), claim 1, and $R^x$ is as defined in respect to formula (IV), as defined above, with a suitable hydride reducing agent, and thereafter, where necessary, carrying out steps (i) to (iii) as described above.

8. A pharmaceutical composition comprising a compound as claimed in any one of claims 1 to 6, or when made by a process according to claim 7, together with a pharmaceutically acceptable carrier or excipient.

9. A pharmaceutical composition as claimed in claim 8, in dosage unit form.

10. A compound as claimed in claim 1, for the use in the treatment atherosclerosis in humans or non-human animals.